# EUROPÄISCHE PATENTANMELDUNG

(11) **EP 1 932 903 A1**
(43) Veröffentlichungstag der Anmeldung: **18.06.2008**
(21) Anmeldenummer: 07123036.1
(22) Anmeldetag: 12.12.2007
(51) Int. Cl.: C12M 1/107, C02F 11/04

(54) **Vorrichtung und Verfahren zum Erzeugen von Biogas**

(30) Priorität: 15.12.2006 DE 102006059769
(71) Anmelder: Polyplan GmbH, 28359 Bremen (DE)
(72) Erfinder: Bruns, Stefan, 27367, Sottrum (DE)
(74) Vertreter: Tappe, Udo

(57) **Zusammenfassung**

Bei einer Vorrichtung zum Erzeugen von Biogas aus organischem Material, insbesondere einem Biogasreaktor (10), mit einer Behandlungskammer (11) für das organische Material läßt sich das Sediment in einem, vorzugsweise stehenden, Gewässer (13) einfach und effizient dann reduzieren, wenn die Behandlungskammer (11) mindestens einen Schwimmkörper (12) aufweist und an ihrer Unterseite offen ist, wobei die Behandlungskammer (11) auf eine Sedimentschicht des Gewässers absenkbar ist.

## Beschreibung

Die Erfindung betrifft eine Vorrichtung zum Erzeugen von Biogas aus organischem Material, insbesondere einen Biogasreaktor, mit einer Behandlungskammer für das organische Material. Außerdem betrifft die Erfindung ein Verfahren zum Behandeln von Sedimenten in, vorzugsweise stehenden, Gewässern.

Aus der CH 662 748 A5 ist eine Vorrichtung zum Auffangen von flüssigen oder gasförmigen Fluiden mit einer schwimmfähigen Auffangvorrichtung bekannt. Im hierfür angedachten Anwendungsbereich von Fäkaliengruben können die aufsteigenden Gase aufgefangen, abgeleitet und genutzt werden. Nachteilig ist hierbei, dass zur Stabilisierung Führungsstangen notwendig sind und lediglich bereits entstandene Biogase aufgefangen werden können. Die Auffangvorrichtung ist nicht geeignet, die Entstehung bzw. Erzeugung von Biogas beispielsweise Sedimenten von Gewässern zu ermöglichen.

Sedimente in stehenden Gewässern sind oftmals sehr reich an organischem Material und aus diesem Grund nicht oder nur sehr aufwendig und damit unter hohen Kosten entsorgbar. Gleichzeitig führt die strengere Gesetzgebung durch das Wasser-Haushalts-Gesetz häufig dazu, daß die Wiedereinbringung des Rückspülwassers oder der Restsedimente nicht genehmigungsfähig ist. Sedimente mit hohem Anteil an organischem Material lagern außerdem auch oft in hohem Maße Schadstoffe mit ein, was die Entsorgung nochmals erschwert. Es ist allerdings unter bestimmten Bedingungen möglich, Sedimente mit hohem Anteil an organischem Material anaerob oder aerob weiter zu mineralisieren (sogenannte Biotechnische Entschlammung). Da das organische Material einen Großteil des in den Sedimenten enthaltenen Wassers bindet, läßt sich das Volumen der Sedimente durch eine Reduzierung des Anteils an organischem Material überproportional reduzieren. Im Falle eines anaeroben Abbaus des organischen Materials läßt sich als Biogas Methan herstellen, welches energetisch verwertet werden kann. In herkömmlichen Fermentern von Biogasanlagen treten allerdings Probleme mit der Sandfraktion in den Sedimenten auf. Aus diesen Grund müßten bei konventionellen Biogasanlagen Vorstufen zur Separation der Sandfraktion vorgesehen werden. Dies führte bei der Verwendung von üblichen Biogasanlagen zu einem unerwünschten Zusatzaufwand.

Das der Erfindung zugrunde liegende Problem ist es, eine Vorrichtung zu Erzeugen, von Biogas aus organischem Material anzugeben, mit der sich einfach und wirkungsvoll eine Reduzierung des Anteils an organischen Materialien in den Sedimenten erzielen läßt. Nach einem anderen Aspekt der Erfindung ist es das zugrunde liegende Problem, ein Verfahren zum Behandeln von Sedimenten in Gewässern anzugeben, mit dem sich einfach und wirkungsvoll der Anteil an organischem Material in dem Sediment reduzieren läßt.

Das Problem wird bei einer Vorrichtung der Eingangs genannten Art dadurch gelöst, daß die Behandlungskammer mindestens einen Schwimmkörper aufweist und an ihrer Unterseite offen ist, wobei die Behandlungskammer auf eine Sedimentschicht des Gewässers absenkbar ist. Nach dem anderen Aspekt der Erfindung wird das Problem mit einem Verfahren der Eingangs genannten Art gelöst, bei dem eine Behandlungskammer mittels eines einen Innenraum aufweisenden Schwimmkörpers schwimmend über die zu behandelnden Sedimente gebracht wird, bei dem die Behandlungskammer mittels Fluten des Innenraumes auf die Sedimente abgesetzt wird, und bei dem das organische Material der Sedimente anschließend in der Behandlungskammer zum Erzeugen von Biogas behandelt wird.

Mit der erfindungsgemäßen Vorrichtung und dem erfindungsgemäßen Verfahren läßt sich der Anteil an organischem Material in den Sedimenten verringern, wodurch die negativen Zehrungseigenschaften der Sedimente auf das Gewässer reduziert werden. Es tritt somit die gewünschte Gewässerverjüngung ein. Gleichzeitig stellt die in den Sedimenten vorhandene Sandfraktion kein Problem dar, weil sie in dem Gewässer verbleiben kann. Wegen der offenen Unterseite der Behandlungskammer wird die Sandfraktion beim Aufschwimmen der erfindungsgemäßen Vorrichtung automatisch aus der Behandlungskammer entfernt und kann somit die erfindungsgemäße Vorrichtung nicht beeinträchtigen. Wegen des Verbleibs der Sandfraktion in dem Gewässer ist ein Wiedereinbringen nicht erforderlich.

Bei einer Weiterbildung der Erfindung weist der Schwimmkörper einen Innenraum auf. Dieser Innenraum kann zum Ablassen der Behandlungskammer auf die Sedimentschicht geflutet und nach durchgeführter Behandlung zum Wiederanheben der Behandlungskammer angeblasen werden.

Eine andere Weiterbildung der Erfindung ist gekennzeichnet durch Umwälzmittel zum Umwälzen von in der Behandlungskammer befindlichen Sedimenten. Auf diese Weise wird eine Behandlung sämtlichen in den Sedimenten vorhandenen organischen Materials erreicht. Als Umwälzmittel eignen sich beispielsweise langgestreckte, sich vertikal erstreckende Mischlanzen. Diese Mischlanzen können mittels Einbringen eines Gases oder mittels Aufspülen mit Wasser das Sediment aufwirbeln und so die gewünschte Umwälzung bewirken. Es ist dabei von Vorteil, wenn die vertikale Erstreckungstiefe der Mischlanzen einstellbar ist. Die Mischlanzen können dann zum Durchmischen in die Sedimente eingeschoben werden. Während der Behandlung ist es dann von Vorteil, wenn die Mischlanzen langsam zurückgezogen werden. Wenn dabei beispielsweise die Mischlanzen über den Fermentationsprozeß von 20 bis 40 Tagen langsam angehoben werden, wird dabei die Sandfraktion wirkungsvoll von dem organischen Material getrennt, so daß zum Schluß nur noch die oben liegende, organisch angereicherte Fermentationsschicht durchmischt wird. Eine wirkungsvolle Ausführungsform der Einstellbarkeit der vertikalen Erstreckungstiefe ergibt sich beispielsweise dann, wenn die Mischlanzen teleskopierbar sind.

Es ist außerdem von Vorteil, wenn eine Lenzpumpe zum Abpumpen von Wasser aus der Behandlungskammer vorgesehen ist. Mittels dieser Lenzpumpe läßt sich der Wassergehalt der Behandlungskammer einstellen.

Bei einer anderen Weiterbildung der Erfindung ist die Behandlungskammer so ausgebildet, daß sie beim Absetzen ein Stück weit in die Sedimente eingelassen wird. Auf diese Weise wird der Behandlungsraum in der Behandlungskammer von dem umgebenden Sediment wirkungsvoll getrennt.

Eine vorteilhafte Ausgestaltung der Erfindung ist gekennzeichnet durch eine der Behandlungskammer zugeordnete Technikeinheit. Es ist dabei von Vorteil, wenn die Technikeinheit schwimmfähig ist. Auf diese Weise kann mittels einer schwimmenden Technikeinheit das erzeugte Biogas genutzt werden. Die Technikeinheit kann sämtliche zum Betrieb der erfindungsgemäßen Vorrichtung erforderlichen technischen Komponenten, wie beispielsweise Steuerung, Pumpen usw. aufweisen. Es ist außerdem von Vorteil, wenn die Technikeinheit eine Gasreinigungsanlage, eine Entnahmestation und/oder ein Blockheizkraftwerk aufweist. Auf diese Weise kann mittels der schwimmenden Technikeinheit das erzeugte Biogas an Ort und Stelle ohne Verluste und großen Transportaufwand genutzt werden. Mittels des Blockheizkraftwerkes kann der Energieeigenbedarf der Technikeinheit durch Eigenverbraucher, wie beispielsweise Kompressoren und Pumpen vollständig bereitgestellt werden. Auf diese Weise kann die erzeugte Energie zu etwa 40% selbst verbraucht werden, während die verbleibenden 60% der von dem Blockheizkraftwerk erzeugten elektrischen Energie mittels einer mobilen Kabelleitung ins Stromnetz eingespeist werden kann. Bei der Verwendung eines solchen Blockheizkraftwerkes bei der Technikeinheit ist es außerdem von Vorteil, wenn Zuführmittel zum Zuführen der Abwärme des Blockheizkraftwerkes in die Behandlungskammer zum Einstellen der Behandlungstemperatur vorgesehen sind. Auf diese Weise kann die Abwärme des Blockheizkraftwerkes zum Heizen der Behandlungskammer zum Einstellen der Reaktortemperatur von etwa 30° bis 40° Celsius für den Fermentationsprozeß genutzt werden.

Eine Weiterbildung des erfindungsgemäßen Verfahrens zeichnet sich dadurch aus, daß die Behandlungskammer nach der Behandlung mittels Anblasen des Innenraumes des Schwimmkörpers angehoben, versetzt und anschließend mit Überlappung mit dem bereits behandelten Bereich mittels Fluten des Innenraumes des Schwimmkörpers wieder auf die Sedimente abgesetzt wird. Durch das Überlappen mit dem bereits behandelten Bereich wird eine Teilfläche des behandelten Sedimentes in den neuen Fermentationsprozeß übernommen, so daß dieser Fermentationsprozeß auf dem neu zu behandelnden Sedimentabschnitt nach dem Versetzten schneller wieder in Gang kommt.

Nachfolgend wird ein Ausführungsbeispiel der Erfindung anhand der Zeichnungen näher erläutert. Es zeigen:
Fig. 1 einen schwimmfähigen Biogasreaktor als Ausführungsbeispiel der Erfindung, und
Fig. 2 den Biogasreaktor von Fig. 2 mit gefluteten Schwimmkörpern.

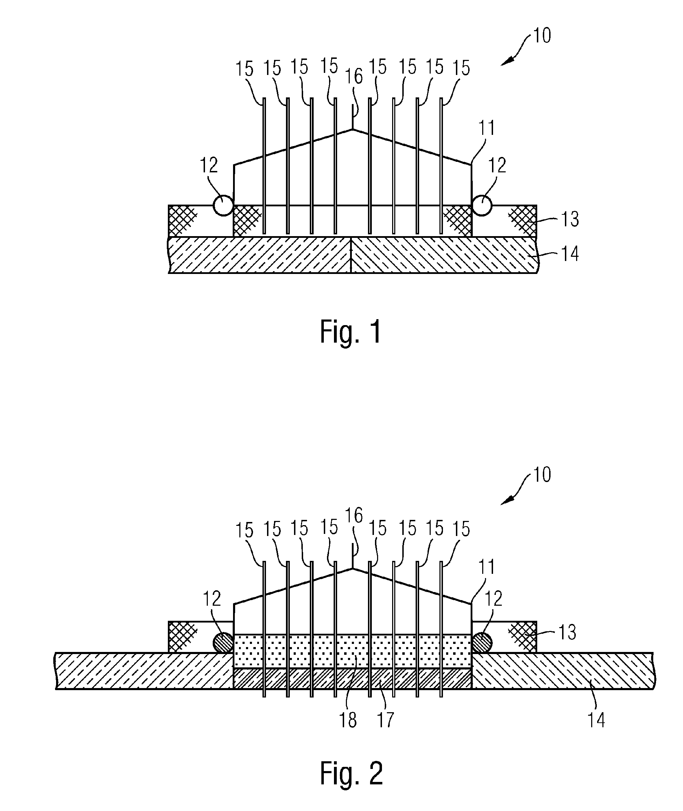

Fig. 1 zeigt eine schematische Darstellung eines Biogasreaktors 10 mit den Erfindungsmerkmalen. Der Biogasreaktor 10 weist eine Behandlungskammer 11, nämlichen einen Fermenter 11 auf, der mit einem Schwimmkörper 12 versehen ist. Der Schwimmkörper 12 ist bei dem gezeigten Ausführungsbeispiel hohl ausgebildet und den Fermenter 11 umgebend und fest mit diesem verbunden angeordnet. In dem gezeigten Zustand ist der Innenraum des Schwimmkörpers 12 mit Luft gefüllt, so daß der Biogasreaktor 10 mit dem Schwimmkörper 12 auf einem Gewässer 13 aufschwimmt. Unter dem Wasser 13 sind schematisch Sedimente 14 dargestellt. Wie sich der Figur weiter entnehmen läßt, befinden sich die nach unten über den Schwimmkörper 12 überstehenden Seitenwände des Fermenters 11 über den Sedimenten 14.

In der Figur ist weiter zu entnehmen, daß mehrere Mischlanzen 15 in dem Fermenter 11 vertikal angeordnet sind. Nicht in der Figur zu sehen ist eine schwimmende Technikeinheit, die mit den Mischlanzen 15 zum Einblasen eines Gases, verbunden ist. Außerdem in der Figur zu sehen ist ein Auslaß 16 für in dem Fermenter 11 produziertes Biogas. Der Auslaß 16 ist mit nicht in der Figur dargestellten Leitungen mit der Technikeinheit verbunden. Die Figur zeigt die Mischlanzen 15 in einem zurückgezogenen Zustand, in dem die Mischlanzen 15 nicht über die Unterseite des Fermenters 11 überstehen.

Fig. 2 zeigt einen Zustand des Biogasreaktors 10, bei dem der Innenraum des Schwimmkörpers 12 zum Absenken des Fermenters geflutet worden ist. Wie sich der Figur entnehmen läßt, sind die unteren Seitenwände durch das Eigengewicht des Biogasreaktors 10 in die Sedimentschicht 14 eingedrückt worden. Der Schwimmkörper 12 liegt auf der Sedimentschicht 14 auf. In dem gezeigten Zustand erstrecken sich die Seitenwände des Fermenters 11 etwa 1 bis 2 Meter tief in die Sedimente 14.

Wie sich der Figur weiter entnehmen läßt, sind die Mischlanzen 15 vertikal nach unten derart verschoben worden, daß sie tief in die Sedimentschicht 14 einstechen. Im Einzelnen reichen die Mischlanzen 15 in dem gezeigten Zustand weiter in die Sedimentschicht als die unteren Seitenwände des Fermenters 11. Mittels der Technikeinheit wird mit geeigneten, nicht in der Figur dargestellten Lenzpumpen der Wasserstand in dem Fermenter 11 auf das gewünschte Maß eingestellt. Gleichzeitig wird mittels nicht in der Figur dargestellter Pumpen der Technikeinheit ein Mischgas in die Mischlanzen 15 eingebracht. Dieses Mischgas strömt aus den unteren Enden der Mischlanzen 15 nach oben und sorgt dabei für ein Durchmischen des Sedimentes 14. Bei diesem Durchmischen erfolgt in einer Fraktionierungszone 17 eine Trennung des organischen Materials von der Sandfraktion. Dieses organische Material schwimmt mit dem eingeleiteten Gas auf und bildet in einem oberen Bereich eine Reaktionszone 18, in der die Biogasproduktion erfolgt.

Das bei der Behandlung erzeugte Biogas wird aus dem Gasauslaß 16 entnommen und der nicht in der Figur gezeigten Technikeinheit zugeführt. Dort kann es mittels einer Gasreinigungsanlage gereinigt und mittels einer Entnahmestation entnommen werden. Es ist aber auch möglich, ein Blockheizkraftwerk vorzusehen. Bei dem gezeigten Ausführungsbeispiel wird das erzeugte Biogas in einem Blockheizkraftwerk verwertet, so daß die Abwärme des Blockheizkraftwerkes der Reaktionszone 18 zum Einstellen der gewünschten Reaktortemperatur von 30° bis 40° Celsius zugeführt werden kann.

Nach der Biogasproduktion wird der Innenraum des Schwimmkörpers 12 angeblasen, so daß der Fermenter 11 wieder aufschwimmt. In diesem Zustand wird der Fermenter 11 ein Stück weit versetzt und durch anschließendes Fluten des Innenraumes des Schwimmkörpers 12 wieder auf das Sediment abgesetzt. Dabei wird eine geringfügige Überlappung mit der bereits behandelten Sedimentfläche 14 gewählt, so daß die Reaktion in der neu zu behandelnden Sedimentfläche 14 schneller in Gang kommt.

### Bezugszeichenliste:

- 10: Biogasreaktor
- 11: Fermenter
- 12: Schwimmkörper
- 13: Wasser
- 14: Sediment
- 15: Mischlanze
- 16: Gasauslaß
- 17: Fraktionierungszone
- 18: Reaktionszone

## Patentansprüche

1. Vorrichtung zum Erzeugen von Biogas aus organischem Material, insbesondere Biogasreaktor (10), mit einer Behandlungskammer (11) für das organische Material, **dadurch gekennzeichnet, daß** die Behandlungskammer (11) mindestens einen Schwimmkörper (12) aufweist und an ihrer Unterseite offen ist, wobei die Behandlungskammer (11) auf eine Sedimentschicht des Gewässers absenkbar ist.

2. Vorrichtung nach Anspruch 1, **dadurch gekennzeichnet, daß** der Schwimmkörper (12) einen Innenraum aufweist, und daß vorzugsweise Mittel zum wahlweisen Fluten oder Anblasen des Innenraumes des Schwimmkörpers (12) vorgesehen sind.

3. Vorrichtung nach einem der Ansprüche 1 oder 2, **gekennzeichnet durch** Umwälzmittel (15) zum Umwälzen von in der Behandlungskammer (11) befindlichen Sedimenten (17, 18), wobei die Umwälzmittel (15) vorzugsweise langgestreckte, sich vertikal erstreckende Mischlanzen (15) aufweisen, und vorzugsweise die vertikale Erstreckungstiefe der, insbesondere teleskopierbaren, Mischlanzen (15) einstellbar ist.

4. Vorrichtung nach einem der vorhergehenden Ansprüche, **gekennzeichnet durch** eine Lenzpumpe zum Abpumpen von Wasser aus der Behandlungskammer (11).

5. Vorrichtung nach einem der vorhergehenden Ansprüche, **dadurch gekennzeichnet, daß** eine, insbesondere schwimmfähige, Technikeinheit der Behandlungskammer (11) zugeordnet ist, daß die Technikeinheit vorzugsweise eine Gasreinigungsanlage, eine Entnahmestation und/oder ein Blockheizkraftwerk aufweist, und daß insbesondere Zuführmittel zum Zuführen der Abwärme des Blockheizkraftwerkes in die Behandlungskammer (11) zum Einstellen der Behandlungstemperatur vorgesehen sind.

6. Verfahren zum Behandeln von Sedimenten in, vorzugsweise stehenden, Gewässern, bei dem eine Behandlungskammer (11) mittels eines einen Innenraum aufweisenden Schwimmkörpers (12) schwimmend über die zu behandelnden Sedimente (14) gebracht wird, bei dem die Behandlungskammer (11) mittels Fluten des Innenraumes auf die Sedimente (14) abgesetzt wird, und bei dem das organische Material der Sedimente (17, 18) anschließend in der Behandlungskammer (11) zum Erzeugen von Biogas behandelt wird.

7. Verfahren nach Anspruch 6, **dadurch gekennzeichnet, daß** die Sedimente (17, 18) mittels Mischlanzen (15) durch Einleiten von Gas durchmischt werden, daß die Mischlanzen (15) vorzugsweise zum Durchmischen in die Sedimente (17, 18) eingeschoben werden, und daß die Mischlanzen (15) insbesondere während der Behandlung langsam zurückgezogen werden.

8. Verfahren nach einem der Ansprüche 6 oder 7, **dadurch gekennzeichnet, daß** die Behandlungskammer (11) beim Absetzen ein Stück weit in die Sedimente (14) eingelassen wird, und daß vorzugsweise der Wassergehalt in der Behandlungskammer (11) mittels einer Lenzpumpe eingestellt wird.

9. Verfahren nach einem der Ansprüche 6 bis 8, **dadurch gekennzeichnet, daß** mittels einer schwimmenden Technikeinheit das erzeugte Biogas genutzt wird, wobei das Biogas vorzugsweise mittels eines Blockheizkraftwerkes genutzt wird, und daß die Abwärme des Blockheizkraftwerkes zum Heizen der Behandlungskammer (11) genutzt wird.

10. Verfahren nach einem der Ansprüche 6 bis 9, **dadurch gekennzeichnet, daß** die Behandlungskammer nach der Behandlung mittels Anblasen des Innenraumes des Schwimmkörpers angehoben, versetzt und anschließend mit Überlappung mit dem bereits behandelten Bereich mittels Fluten des Innenraumes des Schwimmkörpers wieder auf die Sedimente abgesetzt wird.
